# EUROPEAN PATENT APPLICATION

(11) **EP 4 549 563 A1**
(43) Date of publication of application: **07.05.2025**
(21) Application number: 23831862.0
(22) Date of filing: 27.06.2023
(51) Int. Cl.: C12N 9/26, A61K 38/47, A61P 27/02

(54) **HYALURONIDASE HYAL1 VARIANT EXHIBITING ACTIVITY IN NEUTRAL PH**

(30) Priority: 29.06.2022 KR 20220079911; 26.06.2023 KR 20230081667; 26.06.2023 KR 20230082181
(71) Applicant: Odysgen Inc., Daegu 41256 (KR)
(72) Inventor: CHOI, Mal-Gi, Daegu 41256 (KR); LEE, Chang Woo, Daegu 42042 (KR)
(74) Representative: dompatent von Kreisler Selting Werner - Partnerschaft von Patent- und Rechtsanwälten mbB
(86) International application number: PCT/KR2023/008924
(87) International publication number: WO 2024/005502

(57) **Abstract**

The present invention provides: a Hyall variant in which at least one amino acid adjacent to the catalytic amino acids in wild-type human hyaluronidase Hyall, comprising aspartate and glutamate as catalytic amino acids, is substituted with an acidic or polar amino acid; a method for preparing the Hyall variant; a nucleic acid, an expression vector, and a host cell which can be applied to the preparation of the Hyall variant; and a formulation or use of the Hyall variant. In addition, the present invention provides: a Hyall variant in which at least one amino acid adjacent to the catalytic amino acids in the tertiary structure is substituted with a basic amino acid; a method for preparing the Hyall variant; a nucleic acid, an expression vector, and a host cell which can be applied to the preparation of the Hyall variant; and a formulation or use of the Hyall variant. The hyaluronidase Hyall variant of the present invention can effectively decompose hyaluronic acid even at a neutral pH, and thus has the effect of having a high degree of utility.

## Description

### Technical Field

The present disclosure relates to a technology aimed at altering the operating pH range of human hyaluronidase, and specifically to variants of hyaluronidase Hyall, functioning as a catalyst within an acidic to neutral pH range.

### Background Art

Hyaluronidase is an enzyme that hydrolyzes hyaluronic acid. Human hyaluronidases include Hyall, Hyal2, Hyal3, Hyal4, and PH20.

Hyaluronidase PH20, due to its ability to hydrolyze hyaluronic acid in the extracellular matrix, can be used clinically in formulations of antibody therapeutics for subcutaneous injection so as to enhance drug delivery to the subcutaneous tissue. However, the hydrolysis of hyaluronic acid currently relies on one type of enzyme, PH20. That is, among the human hyaluronidases other than PH20, Hyall mainly functions as a catalyst in acidic conditions of pH 3 to 4, limiting its utility. For example, when Hyall is applied to a subcutaneous area with a neutral pH of 7.0 to 7.5, it is difficult to expect the decomposition effect of hyaluronic acids. Additionally, wild-type Hyal2, Hyal3, and Hyal4 have weak activity in the neutral region, rendering them non-utilizable.

Accordingly, the present inventors have conducted studies to improve the utility of wild-type human Hyall, thereby completing the present disclosure.

### Disclosure of Invention

### Technical Problem

An object to solve in the present disclosure is to provide a variant of human Hyall that can effectively hydrolyze hyaluronic acid at neutral pH.

### Solution to Problem

The human hyaluronidase Hyall variant of the present disclosure has Asp129 and Glu131 as catalytic amino acids. In the present disclosure, the amino acid adjacent to the catalytic amino acids in the primary or tertiary structure of Hyall is substituted with an acidic or polar amino acid so that Hyall can perform a catalytic function even at neutral pH.

Additionally, in the tertiary structure of Hyall, any one of the amino acids adjacent to the catalytic amino acids is substituted with a basic amino acid. In particular, the substituted basic amino acid can form an ionic bond with an acidic or polar amino acid adjacent to the catalytic amino acids.

The amino acid adjacent to the catalytic amino acids of Hyall in the primary or tertiary structure is Ala132 or an amino acid located within the loop spanning from Ser76 to Leu98, or adjacent to an acidic or polar amino acid that can influence the activity of the catalytic amino acids in Hyal1's tertiary structure.

Ala132 can be substituted with an acidic or polar amino acid. In particular, the acidic amino acid is Asp or Glu, and the polar amino acid is Ser, Thr, Asn, Gln, or Tyr. Within the loop from Ser76 to Leu98, amino acids adjacent to the catalytic amino acids in the tertiary structure are Ser, Thr, or Pro, and these amino acids are substituted with Asp or Glu.

Meanwhile, other adjacent amino acids that can influence the activity of catalytic amino acids in the tertiary structure of Hyall are the acidic amino acid Asp206 and the polar amino acid Tyr210. The amino acid adjacent to Asp206 or Tyr210 is substituted with the basic amino acid Arg, Lys, or His.

For example, substituting Phe139, located adjacent to Tyr210, with Arg (a basic amino acid) forms an ionic bond between Arg139 and Tyr210. Likewise, when Tyr210, adjacent to Asp206, is substituted with His (a basic amino acid), an ionic bond forms between Asp206 and His210. As a result, the catalytic amino acids can hydrolyze hyaluronic acid even at neutral pH.

Additionally, the present disclosure provides a nucleic acid encoding the hyaluronidase Hyall variant.

Additionally, the present disclosure provides a recombinant expression vector including the nucleic acid.

Additionally, the present disclosure provides a host cell transfected with the expression vector.

Additionally, the present disclosure provides a method for producing hyaluronidase Hyall variant, including culturing of the host cell.

Additionally, the present disclosure provides a hyaluronic acid decomposition agent including the hyaluronidase Hyall variant.

Additionally, the present disclosure provides a drug delivery agent including the hyaluronidase Hyall variant. Additionally, the present disclosure provides a preparation for intravenous injection including the hyaluronidase Hyall variant.

Additionally, the present disclosure provides a preparation for subcutaneous administration including the hyaluronidase Hyall variant.

Additionally, the present disclosure provides an ophthalmological preparation including the hyaluronidase Hyall variant.

### Advantageous Effects of Invention

The hyaluronidase Hyall variant of the present disclosure can effectively decompose hyaluronic acids not only at acidic pH but also at neutral pH, thereby having the effect of a high degree of utility. That is, the hyaluronidase of the present disclosure can decompose hyaluronic acids and deliver drugs using the same even at neutral pH, and can also be used for various uses and preparations such as a preparation for subcutaneous administration, a preparation for intravenous injection, and an ophthalmological preparation. Additionally, the hyaluronidase of the present disclosure may be effectively prepared using the nucleic acid, expression vector, host cell, and method of the present disclosure.

### Brief Description of Drawings

FIG. 1(A) shows the results of an experiment examining the enzyme activity based on pH levels for wild-type Hyall and the Ala132 variants;
FIG. 1(B) shows the results of an experiment confirming the expression of wild-type Hyall and the Ala132 variants in CHO-K1 cells;
FIG. 2(A) shows the results of an experiment confirming the enzyme activity of wild-type Hyall and the Ser77, Thr86, or Pro87 variants across varying pH levels;
FIG. 2(B) shows the results of an experiment confirming the expression of wild-type Hyall and the Ser77, Thr86, and Pro87 variants in CHO-K1 cells;
FIG. 3(A) shows the results of an experiment examining whether variants with two or more substitutions among Ala132, Ser77, and Thr86 exhibit enzyme activity depending on pH;
FIG. 3(B) shows the results of an experiment confirming the expression of variants with two or more substitutions among Ala132, Ser77, and Thr86 in CHO-K1 cells;
FIG. 4 compares the structures of the enzyme active sites of human Hyall and human PH20;
FIG. 5(A) shows the results of an experiment examining whether wild-type Hyall and the Phe139, Tyr210, and Phe139/Ile225 variants exhibit enzyme activity depending on pH;
FIG. 5(B) shows the results of an experiment confirming the expression of wild-type Hyall and the Phe139, Tyr210, and Phe139/Ile225 variants in CHO-K1 cells; and
FIG. 5(C) shows the positions of Phe139, Asp206, Tyr210, and Ile225 adjacent to the catalytic amino acids in the tertiary structure of Hyall.

### Mode for the Invention

Hereinafter, the advantages and features of the present disclosure, as well as the methods to achieve them, will become apparent by referring to the Examples described in detail below and the accompanying drawings. However, the present disclosure is not limited to the Examples disclosed below and can be implemented in various forms. These Examples are merely provided to ensure completeness in disclosing the present disclosure and to fully inform those skilled in the art about the disclosure. The present disclosure is defined only by the scope of the claims.

Throughout this specification, "and/or" includes each and all combinations of at least one of the mentioned constituents. The terminology used herein is for describing embodiments and is not intended to limit the present disclosure. As used herein, singular forms also include plural forms, unless specifically stated otherwise in the context. As used in the specification, "comprises" and/or "comprising" does not exclude the presence or addition of at least one other constituents.

The hyaluronidase Hyall variant (hereinafter referred to as 'Hyal1 variant'), which is an embodiment of the present disclosure, refers to a Hyall variant wherein at least one amino acid adjacent to the catalytic amino acids in the primary or tertiary structure of wild-type human hyaluronidase Hyall including aspartate (e.g., Asp129) and glutamate (e.g., Glu131) as catalytic amino acids is substituted with an acidic or polar amino acid.

In the Hyal1 variant, which is another embodiment of the present disclosure, any one of the amino acids adjacent to the catalytic amino acids in the tertiary structure of wild-type Hyal1 is substituted with a basic amino acid. In particular, the substituted basic amino acid can form an ionic bond with an acidic or polar amino acid adjacent to the catalytic amino acids.

That is, the hyaluronidase Hyal1 variant, which is an embodiment of the present disclosure, corresponds to a variant of wild-type human Hyal1, and by way of substituting the amino acid adjacent to the catalytic amino acids in the primary or tertiary structure with a specific acidic amino acid or polar amino acid, or by way of substituting the amino acid adjacent to the catalytic amino acids in the tertiary structure with a basic amino acid, it is possible for the hyaluronidase Hyal1 variant to effectively decompose hyaluronic acid even at neutral pH, thereby capable of increasing the degree of its utility.

As used herein, the expressions such as "Ala132" or "A132" written in a 3-letter or 1-letter amino acid name followed by a number refer to the amino acid at each corresponding position within the amino acid sequence of SEQ ID NO: 1. For example, "Ala132" and "A132" represent Ala, located at position 132 according to the amino acid sequence of SEQ ID NO: 1. Additionally, as used herein, the variant of wild-type human hyaluronidase also includes a variant in which an amino acid is conservatively substituted at a specific position. In particular, "conservative substitution" refers to a modification of a variant that includes substituting at least one amino acid with an amino acid with similar biological or biochemical properties that does not cause the loss of the biological or biochemical function of the corresponding variant. Specifically, a "conservatively substituted variant" may be a variant that has, for example, at least 80%, preferably at least 90%, more preferably at least 95%, and even more preferably at least 99% sequence homology with the Hyal1 variant consisting of the amino acid sequence selected from the group consisting of the amino acid sequence of SEQ ID NO: 2 to SEQ ID NO: 20 and may be a variant having substantially the same function and/or effect.

The Hyal1 variant, which is an embodiment of the present disclosure, represents hyaluronidase capable of decomposing hyaluronic acids across a broad pH range from acidic to neutral. In this particular one embodiment, the wild-type human hyaluronidase is Hyal1, and Hyal1 consists of the amino acid sequence of SEQ ID NO: 1, and may include at least one amino acid substitution selected from the group consisting of S77D, S77E, T86D, T86E, P87E, A132D, A132E, F139R, Y210H, and F139R/I225D. That is, the variant of the present disclosure, in one embodiment, may include at least one amino acid substitution selected from the group consisting of S77D, S77E, T86D, T86E, P87E, A132D, A132E, F139R, Y210H, and F139R/I225D, based on the wild-type Hyal1 consisting of the amino acid sequence of SEQ ID NO: 1.

In particular, S77D means that Ser in position 77 of SEQ ID NO: 1 is substituted with Asp.

Hyal1 variants are modified forms of wild-type human Hyal1, involving substitutions of specific amino acids, including at least one selected from the group consisting of S77D, S77E, T86D, T86E, P87E, A132D, A132E, F139R, Y210H, and F139R/I225D. In particular, the substitution of F139R/I225D means that the amino acid substitutions of F139R and I225D were made simultaneously.

The amino acid sequence of wild-type human Hyal1 is presented in Table 1 below. As shown in the sequences listed in Table 1, Ala132 is located adjacent to the catalytic amino acids Asp129 and Glu131. In this way, the Hyal1 variant, wherein the amino acid adjacent to the catalytic amino acids is substituted with a specific amino acid, appears to exhibit distinct characteristics compared to those of wild-type Hyal1 while maintaining hyaluronic acid hydrolysis activity. That is, as evidenced by the experimental examples, when Ala132 is substituted with Asp or Glu, the Hyal1 variant can hydrolyze hyaluronic acids even at neutral pH.

That is, by replacing Ala with Asp or Glu at position 132 in the amino acid sequence of wild-type human Hyal1, hyaluronic acid hydrolysis can occur even at neutral pH. However, substituting Ala132 with another similar amino acid in the Hyal1 variant did not yield the same effect. Therefore, it is evident that effective degradation of hyaluronic acids can be achieved, particularly through the utilization of the Hyal1 variant, an embodiment of the present disclosure.

Additionally, Ser77 is located adjacent to the catalytic amino acids in the tertiary structure of Hyal1, and by substituting Ser77 with Asp or Glu, hyaluronic acid can be hydrolyzed at neutral pH. That is, by substituting Ser, at position 77 in the amino acid sequence of wild-type human Hyal1, with Asp or Glu, hyaluronic acid can be hydrolyzed even at neutral pH.

Additionally, Thr86 and Pro87 are located adjacent to the catalytic amino acids in the tertiary structure of Hyal1, and by way of substituting Thr86 with Asp or Glu or substituting Pro87 with Glu, hyaluronic acids can also be hydrolyzed at neutral pH. That is, replacing Thr and Pro at positions 86 and 87, respectively, in the amino acid sequence of wild-type human Hyal1 with Asp or Glu enables the hydrolysis of hyaluronic acid even at neutral pH.

Meanwhile, Asp206 and Tyr210 are located adjacent to the catalytic amino acids in the tertiary structure of Hyal1. In particular, when Phe139, which is adjacent to Tyr210, is substituted with a basic amino acid, it can form an ionic bond with Tyr210, whereas when Tyr210, which is adjacent to Asp206, is substituted with a basic amino acid, it can form an ionic bond with Asp206.

Specifically, when Phe139 is substituted with Arg, the hydroxyl group of Tyr210 exists in a deprotonated state due to the ionic bond between Arg139 and Tyr210, thereby increasing the pKa value of the catalytic amino acid Glu131. Additionally, when Phe139 and Ile225 are substituted simultaneously with Arg and Asp, respectively (F139R/I225D), the positive charge of Arg139 is stabilized by the ionic bond between Arg139 and Asp225. Consequently, Arg139 forms an ionic bond with Tyr210, leading to the deprotonation of the hydroxyl group of Tyr210.

Meanwhile, when Tyr210 is substituted with His, the pKa value of Asp206 decreases due to the ionic bond between Asp206 and His210, and as a result, the pKa value of the catalytic amino acid Glu131 can be increased.

The amino acid sequence of wild-type Hyal1 is described in Table 1, the amino acid sequences of the Ala132 variants in Table 2, the amino acid sequences of the loop variants in Table 3, the amino acid sequences of variants substituted with one or more selected from Ala132 variants or loop variants in Table 4, and the amino acid sequences of Phe139 and Tyr210 variants in Table 5.

**[Table 1]**

| | | |
|---|---|---|
| Wild-type Hyal1 | SEQ ID NO: | Amino Acid Sequence |
| | 1 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |

**[Table 2]**

| Variant s | SEQ ID NO: | Amino Acid Sequence |
|---|---|---|
| A132E | 2 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP |
| | | DFSGLAVIDWEEWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| A132N | 3 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWENWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| A132D | 4 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEDWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| A132Y | 5 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEYWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| A132S | 6 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWESWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| A132H | 7 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEHWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ |
| | | ALCSGHGRCVRRTSHPKALLLLNPASFSIQLTPGGGPLSLRGALSLEDQAQMAVEFKCRC YPGWQAPWCERKSMW |

**[Table 3]**

| Variant s | SEQ ID NO: | Amino Acid Sequence |
|---|---|---|
| S77D | 8 | MAAHLLPICALFLTLLDMAQGFRGPLLPNRPFTTVWNANTQWCLERHGVDVDVSVFDVVA NPGQTFRGPDMTIFYSDQLGTYPYYTPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| S77E | 9 | MAAHLLPICALFLTLLDMAQGFRGPLLPNRPFTTVWNANTQWCLERHGVDVDVSVFDVVA NPGQTFRGPDMTIFYSEQLGTYPYYTPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| T86D | 10 | MAAHLLPICALFLTLLDMAQGFRGPLLPNRPFTTVWNANTQWCLERHGVDVDVSVFDVVA NPGQTFRGPDMTIFYSSQLGTYPYYDPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| T86E | 11 | MAAHLLPICALFLTLLDMAQGFRGPLLPNRPFTTVWNANTQWCLERHGVDVDVSVFDVVA NPGQTFRGPDMTIFYSSQLGTYPYYEPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| P87E | 12 | MAAHLLPICALFLTLLDMAQGFRGPLLPNRPFTTVWNANTQWCLERHGVDVDVSVFDVVA NPGQTFRGPDMTIFYSSQLGTYPYYTETGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ |
| | | ALCSGHGRCVRRTSHPKALLLLNPASFSIQLTPGGGPLSLRGALSLEDQAQMAVEFKCRC YPGWQAPWCERKSMW |

**[Table 4]**

| Variant s | SEQ ID NO: | Amino Acid Sequence |
|---|---|---|
| S77D/ A132E | 13 | MAAHLLPICALFLTLLDMAQGFRGPLLPNRPFTTVWNANTQWCLERHGVDVDVSVFDVVA NPGQTFRGPDMTIFYSDQLGTYPYYTPTGEPVFGGLPQNASLIAHLARTFQDILAAIPAP DFSGLAVIDWEEWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| S77E/ A132E | 14 | MAAHLLPICALFLTLLDMAQGFRGPLLPNRPFTTVWNANTQWCLERHGVDVDVSVFDVVA NPGQTFRGPDMTIFYSEQLGTYPYYTPTGEPVFGGLPQNASLIAHLARTFQDILAAIPAP DFSGLAVIDWEEWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| S77D/ T86D | 15 | MAAHLLPICALFLTLLDMAQGFRGPLLPNRPFTTVWNANTQWCLERHGVDVDVSVFDVVA NPGQTFRGPDMTIFYSDQLGTYPYYDPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| S77D/ T86E | 16 | MAAHLLPICALFLTLLDMAQGFRGPLLPNRPFTTVWNANTQWCLERHGVDVDVSVFDVVA NPGQTFRGPDMTIFYSDQLGTYPYYEPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| S77D/ T86D/ A132E | 17 | MAAHLLPICALFLTLLDMAQGFRGPLLPNRPFTTVWNANTQWCLERHGVDVDVSVFDVVA NPGQTFRGPDMTIFYSDQLGTYPYYDPTGEPVFGGLPQNASLIAHLARTFQDILAAIPAP DFSGLAVIDWEEWRPRWAFNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ |
| | | ALCSGHGRCVRRTSHPKALLLLNPASFSIQLTPGGGPLSLRGALSLEDQAQMAVEFKCRC YPGWQAPWCERKSMW |

**[Table 5]**

| Variant s | SEQ ID NO: | Amino Acid Sequence |
|---|---|---|
| F139R | 18 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEAWRPRWARNWDTKDIYRQRSRALVQAQHPDWPAPQVEAVAQDQFQGAAR AWMAGTLQLGRALRPRGLWGFYGFPDCYNYDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| Y210H | 19 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEAWRPRWAFNWDTKDIYRQRSRALVQAQHPDWPAPQVEAVAQDQFQGAAR AWMAGTLQLGRALRPRGLWGFYGFPDCYNHDFLSPNYTGQCPSGIRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |
| F139R/ I225D | 20 | MAAHLLPICALFLTLLDMAQGFRGPLLPNRPFTTVWNANTQWCLERHGVDVDVSVFDVVA NPGQTFRGPDMTIFYSSQLGTYPYYTPTGEPVFGGLPQNASLIAHLARTFQDILAAIPAP DFSGLAVIDWEAWRPRWARNWDTKDIYRQRSRALVQAQHPDWPAPQVEAVAQDQFQGAAR AWMAGTLQLGRALRPRGLWGFYGFPDCYNYDFLSPNYTGQCPSGDRAQNDQLGWLWGQSR ALYPSIYMPAVLEGTGKSQMYVQHRVAEAFRVAVAAGDPNLPVLPYVQIFYDTTNHFLPL DELEHSLGESAAQGAAGVVLWVSWENTRTKESCQAIKEYMDTTLGPFILNVTSGALLCSQ ALCSGHGRCVRRTSHPKALLLLNPASFSIQLTPGGGPLSLRGALSLEDQAQMAVEFKCRC YPGWQAPWCERKSMW |

These Hyal1 variants are capable of decomposing hyaluronic acid across a broad pH range from acidic to neutral. Additionally, as the Hyal1 variant, which is an embodiment of the present disclosure, can decompose hyaluronic acid within a pH range from acidic to neutral, it is possible to effectively deliver drugs to various sites. This is because when hyaluronic acid in the extracellular matrix is hydrolyzed, the viscosity of hyaluronic acid decreases while the permeability to tissue (skin) increases. Specifically, given that the subcutaneous layer of the skin maintains a neutral pH of approximately pH 7.0 to 7.5, the embodiment of the present disclosure capable of hyaluronic acid decomposition at neutral pH enhances permeability. Consequently, the drug delivery agent, which is an embodiment of the present disclosure, proves highly effective in delivering drugs to various sites, inclusive of the aforementioned variants.

In particular, drugs are components that exhibit pharmacological activity, and are not limited thereto, but may be, for example, eye relaxants, anesthetics, therapeutic antibody agents, anticancer agents, among others. The drugs may consist of a composition along with the variant of the present disclosure. That is, the variant, which is an embodiment of the present disclosure, may exist as a standalone pharmaceutical composition or in conjunction with a drug.

The Hyal1 variant, which is an embodiment of the present disclosure, may be included in a formulation that can be applied to various areas. For example, preparations for subcutaneous administration, intravenous injection, or ophthalmological preparation, which are each an embodiment of the present disclosure, may include the Hyal1 variant, which is an embodiment of the present disclosure. In particular, the ophthalmological preparation may take the form of an eye drop, serving to facilitate the diffusion of anesthetic agents during ophthalmological surgery. Additionally, the subcutaneous administration preparation may specifically entail an injectable formulation, employed for treatment for hyaluronic acid filler complications. Additionally, the intravenous injection preparation may be intended to increase the accessibility of anticancer agents to tumor cells. This is achieved through the transport of the Hyall variant, an embodiment of the present disclosure, via the bloodstream to hydrolyze overexpressed hyaluronic acids on tumor cell surfaces, thereby improving the access to anticancer agents. In this way, besides the therapeutic applications, the Hyall variant, which is an embodiment of the present disclosure, can also be used for cosmetic purposes. Thus, the preparation according to an embodiment of the present disclosure may be pharmaceutical preparations and/or cosmetic preparations.

The dose of the Hyall variant, which is an embodiment of the present disclosure, ranges from 10 ng/mL to 10 mg/mL per subcutaneous injection, preferably falling between 10 ng/mL to 100 µg/mL. Administration may occur once daily or in divided doses, with the dosage typically based on an adult (body weight of 60 kg); however, variations in dosage may occur depending on factors such as body weight and physical conditions. The Hyall variant, which is an embodiment of the present disclosure, is mainly administered parenterally, such as via subcutaneous or intravenous injection, or as an eye drop.

The Hyall variant, which is an embodiment of the present disclosure, can also be formulated with pharmaceutically acceptable additives and prepared in various forms such as injections, eye drops, transdermal patches, etc.

Pharmaceutically acceptable excipients may be applied depending on a number of factors well known to those skilled in the art, for example, the specific physiologically active agents employed, concentrations thereof, stability and intended bioavailability; the disease and disorder or condition being treated; the individual to be treated, age, size, and general conditions thereof, and factors such as nasal, oral, ocular, topical, transdermal, and muscular should be considered, but are not limited thereto. In general, pharmaceutically acceptable excipients used for administration of physiologically active substances other than oral routes of administration include D5W (5% glucose in water), dextrose, and aqueous solutions including physiological salts within 5% of the volume, and in the case of local injections, various injectable hydrogels may be used to enhance the effect and increase its duration. Additionally, pharmaceutically available excipients may also include additional ingredients that can enhance the stability of active ingredients, such as preservatives and antioxidants. The variant, which is an embodiment of the present disclosure, may be formulated by an appropriate method in the relevant field, and may preferably be formulated according to each disease or condition or depending on the ingredients.

Hyall variant, which is an embodiment of the present disclosure, may be stored in a physiological saline solution or lyophilized in an ampoule with the addition of mannitol or sorbitol thereto, and when used for administration, it may be dissolved in physiological saline or similar solutions. In particular, the physiological saline solution may include a buffer, stabilizer, and/or surfactant.

Additionally, the hyaluronic acid decomposition and/or drug delivery method, which is an embodiment of the present disclosure, includes administering the Hyall variant, which is an embodiment of the present disclosure, to mammals including humans, in need of administration. In particular, the administered Hyall variant, which is an embodiment of the present disclosure, may be an effective amount of the variant.

Additionally, the use, which is an embodiment of the present disclosure, is for the preparation of a hyaluronic acid decomposition agent or drug delivery agent of the Hyall variant, which is an embodiment of the present disclosure.

A Hyall variant, which is an embodiment of the present disclosure, can be produced by genetic engineering technology. For example, through genetic manipulation, a fusion gene encoding a fusion protein consisting of a fusion partner and a Hyall variant protein, which is an embodiment of the present disclosure, is prepared, transfected into a host cell, and then expressed in the form of a fusion protein in a host cell. Thereafter, the Hyall variant, which is an embodiment of the present disclosure, may be cleaved and separated from the fusion protein using a proteolytic enzyme or compound to produce the desired protein form. Accordingly, the Hyall variant, which is an embodiment of the present disclosure, can be effectively produced using a nucleic acid, expression vector, host cell, and/or method, which are embodiments of the present disclosure. Specifically, the nucleic acid, which is an embodiment of the present disclosure, encodes a variant, which is an embodiment of the present disclosure. The nucleic acid may be present in cells, cell lysates, or in a partially purified or substantially pure form. The nucleic acid may exist, for example, in the form of DNA or RNA.

Additionally, the expression vector, which is an embodiment of the present disclosure, includes nucleic acid, which is an embodiment of the present disclosure. To express the Hyall variant, which is an embodiment of the present disclosure, the DNA encoding the Hyall variant, which is an embodiment of the present disclosure, is extracted using molecular biology techniques (e.g., PCR amplification, cDNA cloning using a hybrid expressing the Hyall variant, which is an embodiment of the present disclosure), and such DNA may be operably linked to transcription and translation regulatory sequences and inserted into an expression vector so as to prepare an expression vector, which is an embodiment of the present disclosure. In particular, the "operably linked" may mean that the gene encoding the Hyall variant, which is an embodiment of the present disclosure, is ligated into the vector so that the transcription and translation regulatory sequences in the vector can perform the intended function of regulating the transcription and translation of the gene encoding the Hyall variant, which is an embodiment of the present disclosure. The expression vector and the expression regulatory sequences are selected to be suitable for the host cell for expression to be used. The gene encoding the Hyall variant, which is an embodiment of the present disclosure, is inserted into the expression vector by standard methods (e.g., ligation of a gene fragment encoding the Hyall variant, which is an embodiment of the present disclosure, and a complementary restriction enzyme site on the vector, or blunt end ligation in the absence of a suitable restriction enzyme site). The recombinant expression vector has a regulatory sequence that controls the expression of the gene encoding the Hyall variant, which is an embodiment of the present disclosure, in a host cell. The "regulatory sequence" may include a promoter, enhancer, and other expression regulatory elements (e.g., polyadenylation signal) that regulates the transcription or translation of the gene encoding the Hyall variant, which is an embodiment of the present disclosure. In particular, it is certain that the design of the expression vector may vary by selecting different regulatory sequences depending on factors such as the selection of the host cell to be transfected and the expression level of the protein.

Additionally, the host cell, which is an embodiment of the present disclosure, may include a nucleic acid, which is an embodiment of the present disclosure, or an expression vector, which is an embodiment of the present disclosure. The host cell can be selected from the group consisting of, for example, animal cells, plant cells, yeast, E. coli, and insect cells, but is not limited thereto.

The nucleic acid or expression vector is transfected into a host cell. Various techniques are commonly used to introduce exogenous nucleic acid (DNA or RNA) into a prokaryotic or eukaryotic host cell to "transfect" including electrophoresis, calcium phosphate precipitation, DEAE-dextran transfection, lipofection, among others. Various combinations of expression hosts/vectors may be used to express the Hyall variant, which is an embodiment of the present disclosure. Expression vectors suitable for eukaryotic hosts include expression regulatory sequences derived from SV40, bovine papillomavirus, adenovirus, adenoassociated virus, cytomegalovirus, and retrovirus, but are not limited thereto. Expression vectors applicable to bacterial hosts include bacterial plasmids obtained from Escherichia coli (e.g., pET, pRSET, pBluescript, pGEX2T, pUC vector, colE1, pCR1, pBR322, pMB9, and their derivatives), plasmids compatible with a range of hosts (e.g., RP4), phage DNA that can be exemplified by a wide variety of phage lambda derivatives (e.g., λgt10, λgt11, and NM989), and other DNA phages (e.g., M13 and filamentous single-stranded DNA phages). Useful expression vectors for yeast cells are 2 µm plasmids and their derivatives. A useful vector for insect cells is pVL941.

Additionally, the method for preparing a Hyall variant, which is an embodiment of the present disclosure, may include culturing a host cell, which is an embodiment of the present disclosure. When a recombinant expression vector capable of expressing the Hyall variant, which is an embodiment of the present disclosure, is introduced into a mammalian host cell, the variant, which is an embodiment of the present disclosure, is prepared by culturing the host cell for a sufficient period of time to enable expression within the host cell, and preferably, for a sufficient period of time to allow the Hyall variant, which is an embodiment of the present disclosure, to be secreted into the culture medium where the host cell is cultured.

If necessary, the expressed Hyall variant, which is an embodiment of the present disclosure, can be isolated from the host cell and purified to homogeneity. Separation and purification of the Hyall variant, which is an embodiment of the present disclosure, can utilize conventional protein purification methods, such as chromatography. Chromatography methods may be, for example, a combination of at least one selected from affinity chromatography, ion exchange chromatography, or hydrophobic chromatography, but is not limited thereto. In addition to chromatography, filtration, ultrafiltration, salting out, dialysis, etc. may also be used in combination.

Unless otherwise specified, the matters mentioned in relation to the Hyall variant, hyaluronic acid decomposition agent, drug delivery agent, composition, use, preparation, and method, which are embodiments of the present disclosure, are applied equally to one another within the scope of identity unless there is a contradiction.

Hereinafter, the present disclosure will be further described through Examples, Comparative Examples, and Preparation Examples. However, the following Examples and Preparation Examples are provided solely for illustrating the present disclosure, and the contents of the present disclosure are not limited to the following Examples and Preparation Examples.

Hereinafter, the reagents used in the Examples, etc. are commercially available and those of the highest quality were used. Unless otherwise specified, those purchased from Sigma-Aldrich were used.

### <Example 1> Preparation of Hyall variant

The human Hyall gene (Clone ID: hMU005315) was purchased from the Korea Human Gene Bank. The human Hyall gene was amplified by PCR (Bioneer, AllInOneCycler PCR system) using the pCMV-SPORT6-human Hyall plasmid. The amplified gene was inserted into a vector (named pSGHV1 vector), in which the human growth hormone (hGH) and TEV protease cleavage site were removed from the pSGHV0 vector using restriction enzymes XhoI and NotI. In particular, for protein purification using a Ni2+-column, a DNA sequence encoding six His residues was located at the 3'-end of the Hyall cDNA. Hyall variants were also generated using PCR reactor, and the DNA sequences of the produced Hyall variants was confirmed through the DNA sequencing method. Primers used for PCR are as shown in Table 6.

**[Table 6]**

| Hyal1 | SEQ ID NO: | Primer | DNA sequence (5'→3') |
|---|---|---|---|
| WT | 21 | Hyal1-F | CTCGAGGCCACCATGGCAGCCCACCTGCTTCC (*Xho*I site underlined) |
| | 22 | Hyal1-R | GCGGCCGCTCAGTGGTGATGGTGATGATGCCACATGC (*Not*I site underlined) |
| S77D | 23 | S77D-F | CAATTTTCTATAGCGACCAGCT |
| | 24 | S77D-R | AGCTGGTCGCTATAGAAAATTG |
| S77E | 25 | S77E-F | CTATAGCGAGCAGCTGGG |
| | 26 | S77E-R | CCCAGCTGCTCGCTAT |
| T86D | 27 | T86D-F | CTACCCCTACTACGATCCCAC |
| | 28 | T86D-R | CCCCAGTGGGATCGTAGTAG |
| T86E | 29 | T86E-F | CTACCCCTACTACGAGCCCAC |
| | 30 | T86E-R | CCCCAGTGGGCTCGTAGTAG |
| P87E | 31 | P87E-F | CCCTACTACACGGAAACTGG |
| | 32 | P87E-R | GCTCCCCAGTTTCCGTG |
| A132E | 33 | A132E-F | TGGGAGGAATGGCGCCCAC |
| | 34 | A132E-R | GTGGGCGCCATTCCTCCCA |
| A132N | 35 | A132N-F | TGGGAGAACTGGCGCCCAC |
| | 36 | A132N-R | GTGGGCGCCAGTTCTCCCA |
| A132D | 37 | A132D-F | CATCGACTGGGAGGATTGGCG |
| | 38 | A132D-R | CGCCAATCCTCCCAGTCGATG |
| A132Y | 39 | A132Y-F | CATCGACTGGGAGTATTGGCGC |
| | 40 | A132Y-R | GCGCCAATACTCCCAGTCGATG |
| A132S | 41 | A132S-F | CATCGACTGGGAGAGTTGGCG |
| | 42 | A132S-R | CGCCAACTCTCCCAGTCGATG |
| A132H | 43 | A132H-F | CATCGACTGGGAGCATTGGCG |
| | 44 | A132H-R | CGCCAATGCTCCCAGTCGATG |
| F139R | 45 | F139R-F | CACGCTGGGCCAGAAACTGG |
| | 46 | F139R-R | GGTGTCCCAGTTTCTGGCCCA |
| Y210H | 47 | Y210H-F | CCTGACTGCTACAACCATGAC |
| | 48 | Y210H-R | GCTTAGAAAGTCATGGTTGTAGC |
| I225D | 49 | I225D-F | GCCCATCAGGCGACCGTGC |
| | 50 | I225D-R | GTCATTTTGGGCACGGTCGCCTG |

### <Example 2> Confirmation of enzyme activity and expression of Hyall Ala132 variant

CHO-K1 cells (purchased from Korea Cell Line Bank) were transfected with the plasmid containing either the Hyall wild-type or variant gene, as prepared in Example 1, using polyethylenimine (PEI, Sigma-Aldrich) or Lipofectamine 3000 (ThermoFisher) reagents. When CHO-K1 cells grew to a confluence of 90-95% of the area in a 6-well plate, 150 µL of DMEM medium containing 2 µg of plasmid DNA and 150 µL of DMEM medium containing 6 µg of PEI were mixed. After 30 minutes, the PEI-DNA mixture was carefully transferred to a 6-well plate and incubated in a 5% CO2 incubator.

48 hours after the transfection, the cell culture was harvested and centrifuged at 10,000 × g to obtain the supernatant. Enzyme activities of the wild-type Hyall and variants were measured using a substrate-gel assay Specifically, 10% SDS gel containing hyaluronic acids (1.0 mg/mL) were electrophoresed, followed by removal of SDS using a buffer including Triton X-100 (3% Triton X-100, 50 mM Tris, 100 mM NaCl, pH 7.5). Phosphate buffers including 100 mM NaCl, with pH adjusted to pH 4, 5, 6, and 7 using disodium phosphate and monosodium phosphate, were prepared. Each SDS gel was placed in a phosphate buffer of pH 4 to 7, and the enzyme reaction was performed for 4 to 17 hours while shaking at 50 rpm at 37 °C. After the enzyme reaction, hyaluronic acid in the SDS gel was stained with 1.0% Alcian Blue staining. As the purpose of the present disclosure is to examine the enzyme activity of the Hya1 variants at neutral pH, the enzyme activity was evaluated at pH 6 and pH 7, with pH 4 used as a control.

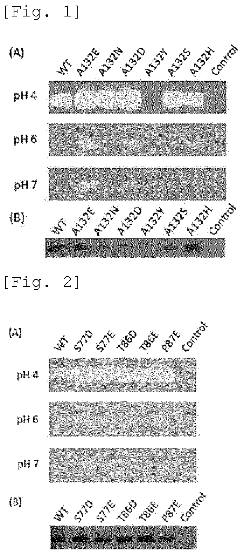

FIG. 1(A) shows the results from an experiment confirming whether Hyall variants exhibit enzyme activity depending on pH. Hyaluronic acid hydrolyzed by the Hyall variant using the substrate-gel assay appears as a white band due to its lack of staining with Alcian Blue. In FIG. 1(A), control represents the control group transfected with only the vector, WT represents wild-type Hyall, and A132E, A132N, A132D, A132Y, A132S, and A132H represent each of the Hyall variants.

As shown in FIG. 1(A), at pH 4, all variants except A132Y hydrolyzed hyaluronic acid and showed enzyme activity; however, at pH 6, A132E, A132D, and A132S variants showed enzyme activity, and at pH 7, only A132E and A132D showed enzyme activity. Among these Hyall variants, A132E showed the highest enzyme activity at pH 7. A132N and A132S, in which the amino acid was substituted with a polar amino acid, showed higher enzyme activity than the wild-type at pH 4, but only A132S showed some enzyme activity at pH 6. The basic amino acid, His, showed enzyme activity at pH 6, but not at pH 7.

Additionally, to assess protein expression in CHO-K1 cells, protein electrophoresis was performed as in the experiment in FIG. 1(A), followed by Western blotting. Specifically, a 10% SDS gel was transferred to a nitrocellulose membrane at 100 V for 1 hour, and then the expression of wild-type Hyall and Ala132 variants were examined using the Hyall monoclonal antibody (1D10, Santa Cruze Biotechnology, Dallas, TX, USA) and the anti-mouse IgG secondary antibody (m-IgGk BP-HRP, Santa Cruze Biotechnology). The results are shown in FIG. 1(B). FIG. 1(B) shows the results of an experiment confirming the expression of the Hyall variants. In FIG. 1(B), Control represents the control group transfected only with the vector, WT represents wild-type Hyall, and A132E, A132N, A132D, A132Y, A132S, and A132H represent each of the variants. As shown in FIG. 1(B), WT, A132E, A132N, A132D, A132S, and A132H variant proteins were detected with the Hyall antibody, but the A132Y variant was not detected. From these results, it can be inferred that the negative result of the A132Y variant shown in FIG. 1(A) is due to a lack of protein expression.

The Hyall variants of the present disclosure hydrolyze hyaluronic acid through general acid-base catalysis. The carboxyl group (-COOH) of the catalytic amino acid Glu131 serves as a proton donor to the hydroxyl leaving group; therefore, the protonation of Glu131 is important for the catalytic reaction. The results in FIG. 1(A) indicate that substituting an amino acid adjacent to the catalytic amino acid Glu131 in the tertiary structure with an acidic amino acid increases the pKa value of Glu131, allowing it to function as a proton donor from acidic to neutral pH. However, even for the same acidic amino acid, A132E showed higher enzyme activity at pH 7 compared to A132D. Therefore, it is evident that in particular, the Hyall variant A132E, which is an embodiment of the present disclosure, can effectively decompose hyaluronic acid even at neutral pH.

### <Example 3> Confirmation of enzyme activity and expression of Hyall loop variant

To assess the enzyme activity of the prepared Hyall loop variants (S77D, S77E, T86D, T86E, and P87E) in the pH range of 4 to 7, CHO-K1 cells were transfected with a plasmid containing the Hyall loop variant gene using PEI or lipofectamine method as described in Example 1. After 48 hours of transfection, cell cultures were collected and centrifuged at 10,000 × g to obtain the supernatant. Enzyme activity of Hyall WT and loop variants present in the supernatant was measured using the substrate-gel assay method, in which enzyme reaction was performed using a phosphate buffer at 37 °C across a pH range of 4 to 7 for 4 to 17 hours, and the amount of the hydrolyzed hyaluronic acid was examined using 1.0% Alcian blue staining. The results are shown in FIG. 2(A). FIG. 2(A) shows the results of an experiment confirming whether Hyall WT and loop variants exhibit enzyme activity depending on pH. In FIG. 2(A), Control represents the control group in which only the vector was transfected, WT represents wild-type Hyall, and S77D, S77E, T86D, T86E, and P87E represent each of the Hyall variants.

As shown in FIG. 2(A), Hyall WT showed enzyme activity by hydrolyzing hyaluronic acid at pH 4, but all of the loop variants (S77D, S77E, T86D, T86E, and P87E) showed enzyme activity across the entire pH range from pH 4 to 7. From these results, it is evident that the Hyall variants, where Ser77, Thr86, and Pro87 in the loop region of wild-type human Hyall were substituted with Asp or Glu, could effectively decompose hyaluronic acid even at neutral pH. In particular, according to the Hyall variant, which is an embodiment of the present disclosure, S77D, S77E, and P87E showed higher enzyme activity than the T86D and T86E variants at pH 7. FIG. 2(B) shows the results of an experiment confirming the expression of the Hyall variants. In FIG. 2(B), it is evident that Hyall WT and the variants were expressed properly in CHO-K1 cells.

### <Example 4> Confirmation of enzyme activity and expression of variants with combination of Hyall Ala132 substitution or loop amino acid substitution

The enzyme activity of Hyall variants with A132E substitution, which exhibited the highest activity among the Ala132 substitutions, combined with at least one of the loop amino acid substitutions, was evaluated across the pH range from pH 4 to 7. To assess the enzyme activity of the prepared Hyall variants (S77D/A132E, S77E/A132E, S77D/T86D, S77D/T86E, and S77D/T86D/A132E) in the pH range of pH 4 to 7, CHO-K1 cells were transfected with a plasmid containing genes for the Hyall variants using PEI or lipofectamine methods as described in Example 1. After 48 hours of transfection, the cell culture was harvested and centrifuged at 10,000 × g to obtain the supernatant. The enzyme activity of the Hyall variants in the supernatant was measured using the substrate-gel assay method, in which enzyme reaction was performed in a phosphate buffer at 37 °C across a pH range from pH 4 to 7 for 4 to 17 hours, and the amount of the hydrolyzed hyaluronic acid was examined using 1.0% Alcian blue staining.

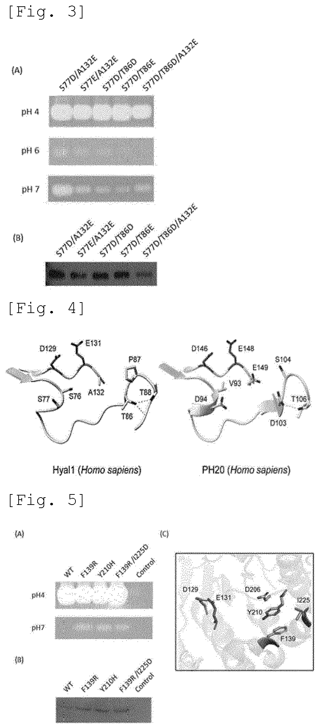

As shown in FIG. 3(A), the variants with double or triple substitutions exhibited enzyme activity by hydrolyzing hyaluronic acid not only at pH 4 but also at pH 7. Particularly, there was a difference in activity level at pH 7, and the S77D/A132E variant showed the highest activity.

FIG. 3(B) shows the results of an experiment confirming the expression of Hyall variants. In FIG. 3(B), it is evident that the Hyall variants were expressed properly in CHO-K1 cells.

### <Example 5> Comparison of protein tertiary structures of human Hyall and PH20

To compare the positions of the substituted amino acids introduced in Hyall in the present disclosure with those in PH20, the tertiary structures of human Hyall and PH20 were compared. The tertiary structure model of human PH20 was created using the Swiss-Model based on the crystal structure of human Hyall (PDB code 2PE4). The structures of human Hyall and PH20 were compared using the PyMol program, and the results are shown in FIG. 4. As shown in FIG. 4, in human Hyall, Ser76 and Ser77, and Thr86 and Pro87 are located near the catalytic amino acid Glu131. In the tertiary structure model of human PH20, Asp94, Asp103, and Glu149 are located adjacent to the catalytic amino acid Glu148.

Therefore, similar to PH20, which exhibits enzyme activity at neutral pH, the Hyall variant of the present disclosure appears to effectively decompose hyaluronic acid even at neutral pH by substituting amino acids, which is adjacent to the catalytic amino acid in the tertiary structure of human Hyall, with an acidic amino acid.

### <Example 6> Confirmation of enzyme activity and expression of F139R and Y210H variants

Another way to increase the pKa value of the catalytic amino acid Glu131 of Hyall is substituting the amino acid adjacent to Glu131 with a basic amino acid, thereby reducing the pKa value of the acidic or polar amino acid that forms an ionic bond with this basic amino acid. In the tertiary structure of Hyall, Phe139, Asp206, Ile225, and Tyr210 are located adjacent to the catalytic amino acids. When the hydrophobic amino acid Phe139 is substituted with Arg, the pKa value of Tyr210 decreases due to the ionic bond between Arg139 and Tyr210, and as a result, the pKa value of the catalytic amino acid Glu131 may increase. Furthermore, substituting hydrophobic amino acids Phe139 and Ile225 with Arg and Asp, respectively, strengthens the positive charge of Arg139 through an ionic bond between Arg139 and Asp225, and as a result, the deprotonation of the hydroxyl group of Tyr210 may be accelerated in the ionic bond between Arg139 and Tyr210 than the F139R variant alone. Additionally, substituting the polar amino acid Tyr210 with the basic amino acid His could decrease the pKa value of Asp206 due to an ionic bond between Asp206 and His210, and as a result, the pKa value of the catalytic amino acid Glu131 may increase.

To assess the enzyme activity of the prepared Hyall variants (F139R, F139R/I225D, and Y210H) in the pH range of pH 4 to 7, CHO-K1 cells were transfected with a plasmid containing the genes for the Hyall variants using PEI or lipofectamine method as described in Example 1. After 48 hours of transfection, the cell culture was harvested and centrifuged at 10,000 × g to obtain the supernatant. The enzyme activity of Hyall WT and the variants present in the supernatant was evaluated using the substrate-gel assay method, in which enzyme reaction was performed using a phosphate buffer at 37 °C over a pH range of pH 4 to 7 for 4 to 17 hours, and the amount of the hydrolyzed hyaluronic acid was examined using 1.0% Alcian blue staining.

In FIG. 5(A), Control represents the control group transfected with the vector alone, WT represents wild-type Hyall, and F139R, Y210H, and F139R/I225D represent each of the Hyall variants. WT exhibited enzyme activity only at pH 4, whereas F139R, Y210H and F139R/I225D variants showed enzyme activity at both pH 4 and 7. The F139R/I225D double variant showed higher activity than the F139R single variant at pH 6, but the single variant and the double variant showed similar enzyme activity at pH 7.

FIG. 5(B) shows the results of an experiment confirming the expression of Hyall variant. In FIG. 5(B), it is evident that the Hyall variants were expressed properly in CHO-K1 cells.

FIG. 5(C) shows the tertiary structure of Hyall with indicated amino acids. Asp206 and Tyr210 are located adjacent to the catalytic amino acid Glu131.

From the results, it is evident that the Hyall variants of the present disclosure can effectively decompose hyaluronic acid not only at acidic pH but also at neutral pH, demonstrating significant utility. That is, as the Hyall variants of the present disclosure can decompose hyaluronic acid even at neutral pH, these Hyall variants could be utilized for drug delivery purposes, as well as for various uses and preparations such as a preparation for subcutaneous administration, a preparation for intravenous injection, and an ophthalmological preparation.

Additionally, from the results above, it is clear that the Hyall variants can be effectively produced using the nucleic acid, expression vector, host cell, and method outlined in the present disclosure.

### <Preparation Example 1> Liquid preparation

One µg each of the Hyall variant proteins prepared in Examples 2 to 6 was dissolved in PBS to make a 1 mL solution. The prepared solution is then filled into an ampoule for injection or sterilized container to produce an injection solution or eye drops. Thus preparation may be used as a hyaluronic acid decomposition agent, drug delivery agent, etc. In particular, injectable solutions can serve as preparations for subcutaneous administration or intravenous injection, and eye drops may be used as ophthalmological preparations.

### References

1. Pace CN, Grimsley GR, Scholtz JM. Protein ionizable groups: pK values and their contribution to protein stability and solubility. J Biol Chem. 2009; 284(20) :13285-13289.
2. Reitinger S, Mullegger J, Greiderer B, Nielsen JE, Lepperdinger G. Designed human serum hyaluronidase 1 variant, HYAL1DeltaL, exhibits activity up to pH 5.9. J Biol Chem. 2009; 284(29):19173-19177.

### [Sequence Listing Free Text]

Attached as an electronic file.

## Claims

1. A hyaluronidase Hyall variant exhibiting activity at neutral pH.

2. The hyaluronidase Hyall variant of claim 1, wherein the hyaluronidase Hyall variant comprises aspartate (Asp) and glutamate (Glu) as catalytic amino acids in wild-type human hyaluronidase Hyall consisting of an amino acid sequence of SEQ ID NO: 1,
wherein at least one amino acid adjacent to the catalytic amino acid(s) in its primary structure or tertiary structure thereof is substituted with an acidic or polar amino acid, or
at least one amino acid adjacent to the catalytic amino acid(s) in its tertiary structure thereof is substituted with a basic amino acid.

3. The hyaluronidase Hyall variant of claim 2, wherein the hyaluronidase Hyall variant comprises at least one amino acid substitution selected from the group consisting of S76D, S77D, T86D, P87E, A132D, and A132E in wild-type human hyaluronidase Hyall.

4. The hyaluronidase Hyall variant of claim 2, wherein the substituted basic amino acid forms an ionic bond with the acidic or polar amino acid adjacent to the catalytic amino acids.

5. The hyaluronidase Hyall variant of claim 2, wherein the hyaluronidase Hyall variant comprises an amino acid substitution selected from the group consisting of F139R, Y210H, and F139R/I225D, and wherein the F139R/I225D means that F139R and I225D are substituted simultaneously.

6. A nucleic acid encoding the hyaluronidase Hyall variant of claim 1.

7. A recombinant expression vector comprising the nucleic acid of claim 6.

8. A host cell transfected with the expression vector of claim 7.

9. A method for preparing a hyaluronidase Hyall variant comprising culturing the host cell of claim 8.

10. A hyaluronic acid decomposition agent comprising the hyaluronidase Hyall variant of claim 1.

11. A drug delivery agent comprising the hyaluronidase Hyall variant of claim 1.

12. A preparation for subcutaneous administration comprising the hyaluronidase Hyall variant of claim **1.**

13. A preparation for intravenous injection comprising the hyaluronidase Hyall variant of claim 1.

14. An ophthalmological preparation comprising the hyaluronidase Hyall variant of claim 1.
